# EUROPEAN PATENT SPECIFICATION

(11) **EP 2 505 664 B1**
(45) Date of publication and mention of the grant of the patent: **03.12.2014**
(21) Application number: 11160484.9
(22) Date of filing: 30.03.2011
(51) Int. Cl.: C12Q 1/68

(54) **Method and means for distinguishing malignant from benign tumor samples, in particular in routine air dried fine needle aspiration biopsy (FNAB)**
Verfahren und Mittel zur Unterscheidung von gut- und bösartigen Tumorproben, insbesondere bei der FNAB
Procédé et moyens pour distinguer des échantillons de tumeurs malignes des échantillons de tumeurs bénignes, en particulier dans la biopsie par aspiration à l'aiguille séchée à l'air

(43) Date of publication of application: 03.10.2012
(73) Proprietor: Universität Leipzig, 04109 Leipzig (DE)
(72) Inventor: Paschke, Ralf, Prof. Dr., 04416 Markleeberg (DE); Eszlinger, Markus, Dr., 04420 Markranstädt (DE)
(74) Representative: Kailuweit & Uhlemann Patentanwälte Partnerschaft mbB

(56) References cited:
- WILLIEFORD MOSES ET AL: "Molecular Testing for Somatic Mutations Improves the Accuracy of Thyroid Fine-needle Aspiration Biopsy", WORLD JOURNAL OF SURGERY ; OFFICIAL JOURNAL OF THE INTERNATIONALSOCIETY OF SURGERY/SOCIÉTÉ INTERNATIONALE DE CHIRURGIE, SPRINGER-VERLAG, NE, vol. 34, no. 11, 12 August 2010 (2010-08-12), pages 2589-2594, XP019842866, ISSN: 1432-2323
- CANTARA SILVIA ET AL: "Impact of proto-oncogene mutation detection in cytological specimens from thyroid nodules improves the diagnostic accuracy of cytology.", THE JOURNAL OF CLINICAL ENDOCRINOLOGY AND METABOLISM MAR 2010 LNKD- PUBMED:20130073, vol. 95, no. 3, March 2010 (2010-03), pages 1365-1369, XP002654265, ISSN: 1945-7197
- CHEUNG CAROL C ET AL: "Analysis of ret/PTC gene rearrangements refines the fine needle aspiration diagnosis of thyroid cancer", JOURNAL OF CLINICAL ENDOCRINOLOGY AND METABOLISM, vol. 86, no. 5, May 2001 (2001-05), pages 2187-2190, XP002654266, ISSN: 0021-972X
- NIKIFOROV YURI E ET AL: "Molecular testing for mutations in improving the fine-needle aspiration diagnosis of thyroid nodules.", THE JOURNAL OF CLINICAL ENDOCRINOLOGY AND METABOLISM JUN 2009 LNKD- PUBMED:19318445, vol. 94, no. 6, June 2009 (2009-06), pages 2092-2098, XP002654267, ISSN: 1945-7197
- MAZEH H ET AL: "Development of a microRNA-based molecular assay for the detection of papillary thyroid carcinoma in aspiration biopsy samples", THYROID 20110201 MARY ANN LIEBERT INC. USA LNKD- DOI:10.1089/THY.2010.0356, vol. 21, no. 2, 1 February 2011 (2011-02-01), pages 111-118, XP002654268, ISSN: 1050-7256
- ESZLINGER M ET AL: "Molecular fine-needle aspiration biopsy diagnosis of thyroid nodules by tumor specific mutations and gene expression patterns", MOLECULAR AND CELLULAR ENDOCRINOLOGY 2010 ELSEVIER IRELAND LTD IRL LNKD- DOI:10.1016/J.MCE.2010.01.010, vol. 322, no. 1-2, June 2010 (2010-06), pages 29-37, XP002654269, ISSN: 0303-7207
- NIKIFOROVA MARINA N ET AL: "Molecular diagnostics and predictors in thyroid cancer.", THYROID : OFFICIAL JOURNAL OF THE AMERICAN THYROID ASSOCIATION DEC 2009, vol. 19, no. 12, December 2009 (2009-12), pages 1351-1361, ISSN: 1557-9077

## Description

The invention as disclosed in claim 1 concerns a method for distinguishing malignant from benign tumors in routine samples, in particular in routine air dried fine needle aspiration biopsy (FNAB) in particular of thyroid nodules.

Thyroid nodules are frequent clinical findings. Their reported prevalence varies from 3-76% depending on the screening method and the population evaluated. However, the incidence of thyroid cancer is low. The annual incidence in areas not affected by nuclear fall out has been reported to range between 1.2 - 2.6 cases per 100.000 in men and 2.0 - 3.8 cases per 100,000 in women with higher incidences in countries like Sweden, France, Japan and USA. Therefore, patients with thyroid nodules require evidence based strategies for the differential diagnosis and risk stratification for malignancy.
Fine-needle aspiration biopsy (FNAB) is the most sensitive and specific tool for the differential diagnosis of thyroid malignancy. Some limitations of FNAB can be overcome by the molecular analysis of FNAB. The preoperative FNAB can reduce the number of surgeries for thyroid nodules to 10% as compared to a strategy without FNAB use with a concomitant increase of thyroid malignancies from 3.1 (without FNAB) to 34% (with FNAB). Under optimal conditions, 60-80% of the biopsied nodules can be classified as benign by cytology and 3.5-5% are classified as malignant. However, the ratio of malignant/benign results for patients undergoing resection of thyroid nodules is still 1 : 15 in Germany or 1 : 7 in Italy thus resulting in a high number of "diagnostic" thyroid surgeries (among the 110,000 annual thyroid surgeries in Germany). Besides other reasons like the selection of suspicious nodules for FNAB by ultrasound malignancy criteria this unsatisfactory situation is mainly due to some limitations of this FNAB focused strategy like the difficulty to determine the rate of false negative cytologies since a nodule diagnosed as benign by FNAB is usually managed conservatively and especially because 10 - 20% of the FNAB samples are classified as follicular proliferation/indeterminate which cannot distinguish between follicular adenoma (FA), adenomatoid hyperplasia (AH), follicular thyroid carcinoma (FTC), and follicular variant of papillary thyroid carcinoma (fvPTC). Therefore, patients with this cytologic finding currently have to undergo (diagnostic) surgery, which will detect thyroid malignancy in about 20% of these patients. This means that 80% of the thyroid FNAB samples that were classified as follicular proliferation/indeterminate lesion by cytology will undergo diagnostic (unnecessary) thyroidectomy. Thus, the follicular proliferation category is the most problematic FNAB category.

In recent years immunohistologic markers like Galectin-3, HBME-1, Fibronectin-1, CITED-1, Cytokeratin-19 have been investigated in order to improve the differential diagnosis between benign and malignant thyroid nodules. However, they have barely been adopted in daily routine diagnostics, mainly because of different methods used and because these markers show prominent overlap between FA and differentiated thyroid carcinomas. Currently, no single cytochemical (or genetic) marker is specific and sensitive enough to reliably further specify or replace the morphologic diagnosis of follicular lesion or suspicious for neoplasm.

US 7,319,011 B2 describes that Follicular thyroid adenoma (FTA) can be distinguished from follicular thyroid carcinoma (FTC) by comparing amount of an expression product of at least one gene selected from the group consisting of DDIT3, ARG2, ITM1, Clorf24, TARSH, and ACOl in a test follicular thyroid specimen to a normal control.

US 7,670,775 B2 discloses methods of identifying malignant thyroid tissue comprising testing a thyroid tissue sample for the expression of at least two genes chosen from CCND2, PCSK2, and PLAB.

US 6,723,506 B2 describes the molecular characterization of PAX8-PPAR1 molecules for detection and treatment of certain tumors, particularly thyroid follicular carcinomas.

US 7,378,233 B2 describes the T1796A mutation of the BRAF gene that was detected in 24 (69%) of the 35 papillary thyroid carcinomas examined. Further, the T1796A mutation was detected in four lung cancers and in six head and neck cancers but not in bladder, cervical, or prostate cancers.

MicroRNAs (miRNAs or miR) are short ribonucleic acid molecules that are post-transcriptional regulators that bind to complementary sequences on target messenger RNA transcripts (mRNAs), usually resulting in translational repression and gene silencing. miR-221 is known to be up-regulated in PTC (Pallante P et al. 2006. Endocr Relat Cancer 13(2):497-508; He H et al. 2005 PNAS 102(52):19075-19080, Mazeh et al. 2011. Thyroid 21(2): 111- 118).

With the discovery of somatic mutations for 42% of PTCs and 65% of FTCs new perspectives for the classification and diagnosis of thyroid tumors in addition to histology have emerged. Molecular testing for somatic mutation has become an immediate and currently the most promising future approach for molecular FNAB diagnosis which will allow a further discrimination of the follicular proliferation/indeterminate and suspicious FNAB categories, to reduce the number of diagnostic thyroid surgeries and the rate of false negative cytologies. Nearly all of the somatic mutations have been tested for their applicability in FNAB diagnosis in different settings in the recent years. Most studies have analyzed one mutation e.g. *BRAF* or *RET*/*PTC* (e. g. Cheung C.C. et al. 2001. J Clin Endocrinol Metab 86(5):2187-2190). Four studies have analyzed several mutations (*BRAF, RAS, RET*/*PTC* and *PAX8*/*PPARg*) (Moses W et al. 2010 World J Surg 34(11):2589-2594; Cantara S et al. 2010 J Clin Endocrinol Metab 95(3):1365-1369; Nikiforov YE et al. 2009 J Clin Endocrinol Metab 94(6):2092-2098; Ohori NP et al. 2010 Cancer Cytopathol 118(1):17-23).

Eszlinger M and Paschke R. 2010 (Mol Cell Endocrinol. 2010 Jun 30;322(1-2):29-37) summarize the molecular genetic techniques to study fine needle aspiration biopsy (FNAB) samples of thyroid tumors. Most studies analyzing one or more mutations (especially the studies investigating the rearrangements) in FNAB material use a separate part of the same FNAB sample fresh or after storage in frozen form for cytology and molecular analysis (e.g. left over cells in the needle bevel plus the needle washing, or a third or part of the total FNAB material obtained, see for instance Cheung et al. 2001; Moses W et al. 2010; Cantara S et al. 2010 and J; Nikiforov YE et al. 2009 as cited above). Others do not use the same FNAB sample for both morphologic and molecular analyses but performed an additional FNAB which can increase the likelihood of generating contradictory results even more.

Only some studies that investigated only BRAF with genomic DNA extraction used the FNAB material from routine air dried or ethanol fixed FNAB smears (e. g. Girlando S, et al. 2010 Int J Surg Pathol 18(3):173-176 and Kim JW et al. 2010. J. Clin. Endocrinol. Metab. 95(8): 3693-3700).

Extraction of RNA from air dried stained cells of cytology FNAB smears for the RT-PCR amplification of *RET*/*PTC* and *PAX8*/*PPARG* rearrangements has up to date not been reported and has been judged to be not feasible (Nikiforova MN, Nikiforov YE 2009, Thyroid 19(12):1351-1361).

Thus a goal of the invention as disclosed in claim 1 is to provide a method for distinguishing malignant from benign tumor samples to facilitate tumor diagnosis in routine samples and to further reduce the number of diagnostic surgeries and the rate of false negative cytologies.

This goal is solved by the invention as disclosed in claim 1 in a first aspect by providing a method for distinguishing malignant from benign tumor samples, in particular of the thyroid, by
a.) performing a RNA extraction in an air dried and/or fixed fine needle aspiration biopsy (FNAB) sample, wherein RNA isolation is performed after cytological fixation and staining of the air dried and/or fixed fine needle aspiration biopsy (FNAB) sample,
b.) analyzing the presence ofgene-rearrangements and/or differential expression of miRNA in the isolated RNA, wherein the presence of a gene-rearrangement and/or the differential expression of miRNA is indicative for a malignant tumor.

An air dried fine needle aspiration biopsy (FNAB) sample is a sample obtained by routine FNAB. FNAB is sometimes also referred to as fine needle aspiration cytology (FNAC). The sample is taken by inserting a thin, hollow needle (preferably ranging from 22 to 27 gauge - commonly, 25 gauge) into the tumor mass. As the name indicates, the biopsy technique uses aspiration to obtain cells and fluid from the tumor mass. The needle is gently moved back and forth through the lesion several (e.g. 3-6) times in different directions to obtain a representative sample of tissue. A representative sample preferably contains at least 6 groups comprising 6 to 8 thyroid cells each. The FNAB sample contains at least 30 thyroid cells and preferably a maximum of 200, more preferably up to 150 thyroid cells, even more preferably up to 100 thyroid cells.

FNAB are very safe, minor surgical procedures. Local anaesthesia is not routinely used for FNAB. If needed, a small amount (0.5 to 1.0 ml) of 1% lidocaine without epinephrine can be infiltrated locally to produce a skin wheal only, in order not to obscure the nodule.

Preferably the obtained FNAB material is directly expelled onto a glass microscope slide. A thin smear is prepared by using the second glass slide to gently press down and draw out the material to a feathered edge. The smear is air dried or fixed immediately preferably in 95% alcohol or other commercially available cytological spray fixative. The slide is stained with a common histological dye, like Papanicolaou stain or Mai Grünwald stain, and assigned a cytology code, e.g. C1 - insufficient material to make a diagnosis; C2 - benign; C3 - indeterminate / follicular proliferation; C4 - suspicious; C5 - malignant.

Surprisingly the air dried or fixed FNAB sample, preferably a routine smear obtained and stained like described above, can be used in the method according to the invention as disclosed in claim 1 for RNA extraction and analysis of gene rearrangements and/or miRNA.

Thus, RNA isolation in the method according to the invention as disclosed in claim 1 is performed after cytology analysis (thus after fixation and staining) of the air dried fine needle aspiration biopsy (FNAB) sample.

This has several advantages:
a.) the cytology analysis and analysis of gene rearrangements (and optionally further molecular analysis) is performed in exactly the same sample, which avoids inconsistent results that results from divergent samples and leads to a direct correlation between cytology and molecular analysis,
b.) fewer material is needed,
c.) integrated and focussed molecular diagnostics is performed only for those (same) FNAB samples which the cytopathologist cannot decide based on cytology criteria,
d.) no need to prepare part of or further FNAB material for RNA preservation,
e.) no need to store part of the FNAB material or additional FNAB material until completion of cytologic diagnosis to then select those stored samples with indeterminate cytology reports for further molecular analysis,
f.) no second FNAB for molecular diagnostics and thus less burden for the patient,
g.) lower total diagnostic cost due to spared unnecessary parallel morphologic and molecular diagnostics (and lower total cost due to spared surgeries).

Alternatively to preparing a smear, the FNAB material obtained is a processed by liquid based cytology. Unlike a traditional FNAB smear, where the cells are placed directly on a microscope slide, in liquid based cytology the FNAB sample is placed into a preservative fluid (also fixing the cells), preferably alcohol, e. g. methanol or ethanol-based. The vial is then sent to the laboratory for further processing e.g. by the T2000 automated processor according to the manufacturer's recommendations. Red blood cells in the FNAB sample are preferably deleted and the thyroid cells are collected, preferably by centrifugation, and applied to a carrier, preferably a slide. The FNAB sample can then be dried, stained and examined in the same manner as a traditional smear by a cytologist.

The same FNAB sample obtained and stained like described above by liquid based cytology can be used in the method according to the invention as disclosed in claim 1 for RNA extraction and analysis of gene rearrangements, point mutations and/or miRNA. Alternatively, left over cells not used for making the first slide can be used in the method according to the invention as disclosed in claim 1 for RNA extraction and analysis of gene rearrangements, miRNA and/or for the detection of point mutations.

Preferably rearrangements of *RET*/*PTC* and/or *PAX8*/*PPARG* (paired box 8/peroxisome proliferator-activated receptor gamma) are detected in the isolated RNA, preferably by reverse-transcribing the isolated mRNA into cDNA and subsequent PCR-analysis (preferably a Real-time PCR) with specific primers and/or oligonucleotide probes that allow the detection of the rearrangements. Primers and oligonucleotide probes are known from the state of the art.

Alternatively the rearrangements are detected by performing sequencing, in particular Pyrosequencing, on the cDNA obtained. Pyrosequencing is a method of DNA sequencing (determining the order of nucleotides in DNA) based on the "sequencing by synthesis" principle. It differs from Sanger sequencing, in that it relies on the detection of pyrophosphate release on nucleotide incorporation, rather than chain termination with dideoxynucleotides (see also Ronaghi M. 2001. Genome Research 11 (1): 3-11).

The RNA extracted contains in particular messenger RNA (mRNA) and miRNA. MicroRNAs (miRNAs) are short ribonucleic acid molecules (about 20-30 nucleotides long) that are post-transcriptional regulators that bind to complementary sequences on target messenger RNA transcripts (mRNAs), usually resulting in translational repression and gene silencing.

Preferably the following miRNA (miR) are detected: miR-21, miR-181, miR-182, miR-187, miR-221, and/or miR-222.

The analysis of miRNA is preferably performed quantitatively. Methods for specific RNA detection are known. The detection is preferably done with primers and/or oligonucleotide probes hybridising to the RNA or after reverse transcription to the corresponding cDNA. The oligonucleotide probes might be labelled or part of a microarray (e. g. a miRNACHIP). In the latter case the RNA isolated is preferably labelled (e. g. biotinylated) and incubated with the microarray.

Preferably, in the method according to the invention as disclosed in claim 1 RNA, in particular mRNA and miRNA, and genomic DNA are extracted simultaneously.

The extracted DNA is preferably used to detect point mutations, in particular in DNA encoding BRAF, N-, K-, and/or HRAS. Commercial assays for the detection of BRAF, KRAS and NRAS mutations can be used.

Preferably mutations selected from the following list are detected:
- BRAF: V600E, K601E (protein), as well as T1796A and T1799A (DNA),
- NRAS: any mutation in codon 61,
- KRAS: any mutation in codon 12 and codon 13,
- HRAS: any mutation in codon 61.

After PCR amplification point mutations are preferably detected with high-resolution-melting (HRM) analysis or Pyrosequencing. HRM is more sensitive than Sanger sequencing. The advantages of pyrosequencing are a high sensitivity and more objective results regarding the mutational status since both qualitative and quantitative information are given.

The presence of a gene-rearrangement and/or the differential expression of miRNA is indicative for a malignant tumor.

In particular, the presence of RET/PTC and/or PAX8/PPARG rearrangements and/or mutations in genes encoding BRAF, N-, K-, and/or HRAS and/or differential expression of miRNA in the sample classifies the tumor as malignant. Differential expression means that the expression is higher or lower than in healthy tissue, in particular of the thyroid.

The detection of any of the point mutations or rearrangements or a differential expression of miRNA gives an indication for total thyroidectomy including central lymph node compartment dissection whereas their absence would argue for follow up.

Another aspect of the invention as disclosed in claim 1, is the use of a kit for carrying out the method of the invention as disclosed in claim 1.

This kit preferably contains at least one of the following components:
i. Buffer for nucleic acid extraction,
ii. Primer (e. g. oligo-dT, random hexamer, target specific reverse transcription primers), buffers and RNA-dependent-DNA-polymerase for Reverse transcription (RT),
iii. Primers (e.g., PCR primers and/or sequencing primers) and optionally oligonucleotide probes (preferably single or dual Fluorescence-labeled), Polymerase (preferably tag polymerase or another thermostable DNA-dependent DNA polymerase) and buffers for PCR on cDNA to detect gene rearrangements, in particular RET/PTC and/or PAX8/PPARG gene rearrangements preferably be real-time PCR or pyrosequencing,
and optionally:
iv. Primers (e.g., PCR primers and/or sequencing primers and optionally oligonucleotide probes), Polymerase (preferably tag polymerase or another thermostable DNA-dependent DNA polymerase) and buffers for PCR on genomic DNA to detect point mutations, in particular in genes encoding BRAF, N-, K-, and/or HRAS and/or
v. oligonucleotide probes (preferably single or dual Fluorescence-labeled) for the detection of miRNA.

The primers for PCR and the oligonucleotide probes, in particular used for real time PCR or detection of the miRNA, are oligonucleotides (preferably with a length of 15 to 25 nucleotides) that are complementary to the target sequence (nucleic acid sequence to be detected) and specifically hybridize thereto by complementary base paring. The oligonucleotide probes are preferably labeled, e. g. with dyes (in particular fluorescent dyes), haptens (such as biotin or digoxigenin) or radioactively.

Alternatively the oligonucleotide probes are part of a microarray, e. g. a miRNACHIP. In this case the RNA isolated is preferably labelled (e. g. biotinylated) and incubated with the microarray.

Thus, in this case the kit preferably contains additionally or alternatively at least one of the following components:
i. Buffer for nucleic acid extraction,
ii. Reagents for labeling RNA and/or DNA, in particular fluorescent dyes,
iii. Hybridization buffers,
iv. Washing buffers.

The invention as disclosed in claim 1 is further illustrated by the following figures and examples without being limited to these.
**Figure 1** shows the results of Thyroglobulin (TG) mRNA and SCARNA17 expression analysis in comparison to the cellularity of the samples. (++ = 20 - 50 thyroid epithelial cells, +++ > 50 thyroid epithelial cells).
**Figure 2** shows the results of miR-221 expression analysis in PTC versus goiter. miR-221 (normalized to SCARNA17) shows a significantly increased expression in PTC versus goiter (p<0.001).
**Figure 3** shows the results of quantification of miRNA housekeeping RNA (RNU6B).
**Figure 4** shows the results of quantification of miRNA miR-21.

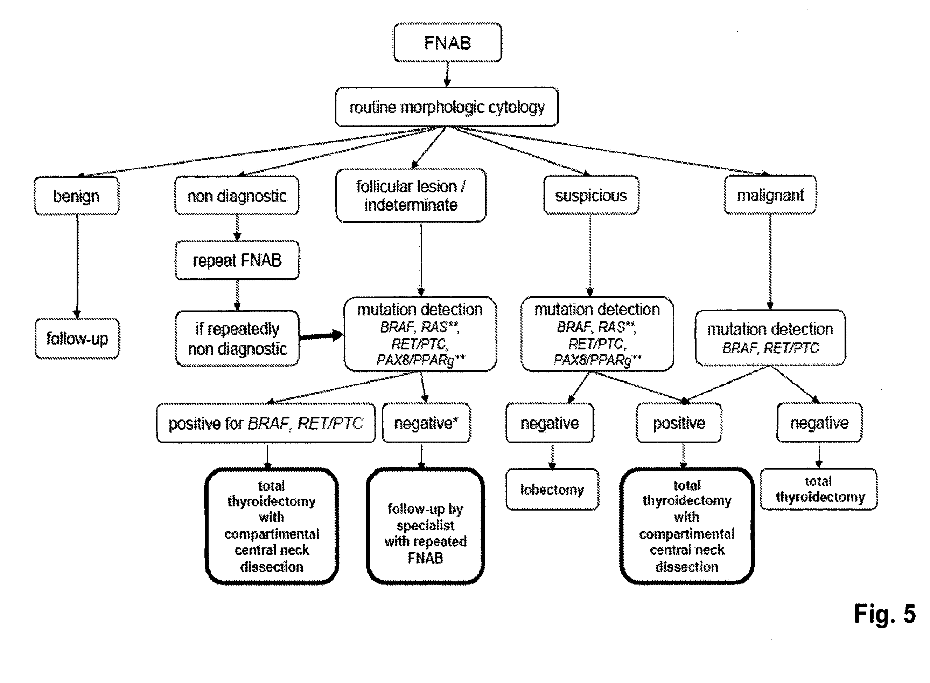

**Figure 5** shows the additional clinical consequences of the molecular diagnostic provided by the invention as disclosed in claim 1 in bold.
   * optional further diagnosis by miRNA markers,
   ** if positive for RAS mutation or PAX8/PPARg rearrangement lobectomy in general justified.
**Figure 6** shows the results of RPL27 and TG mRNA expression analysis in samples process.
**Figure 7** shows the results of a screening for BRAF mutations by HRM.

### 1. Setting-up DNA, mRNA, and miRNA extraction methods from routine FNAB

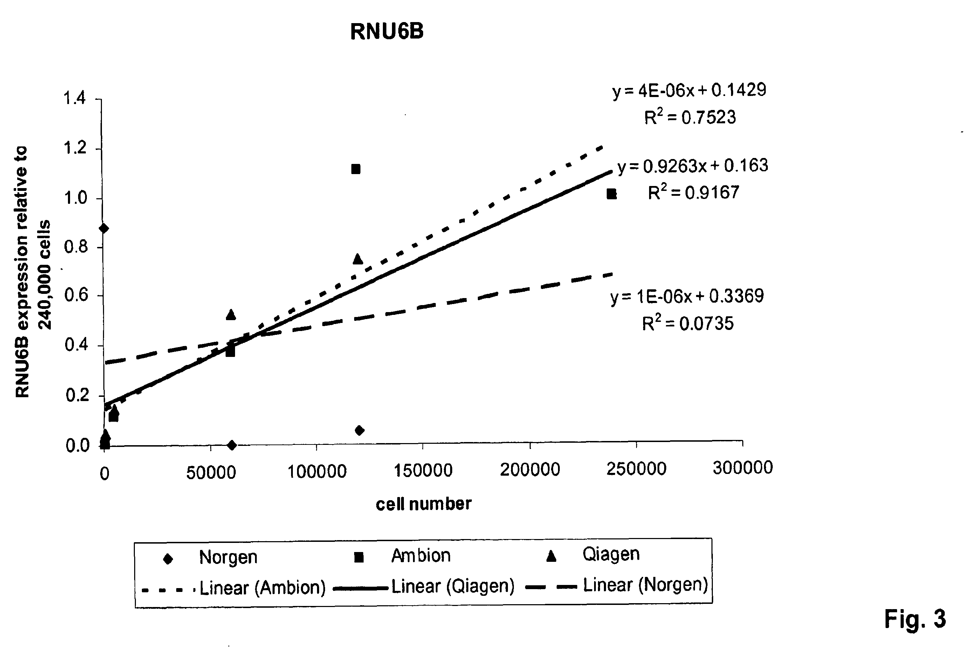

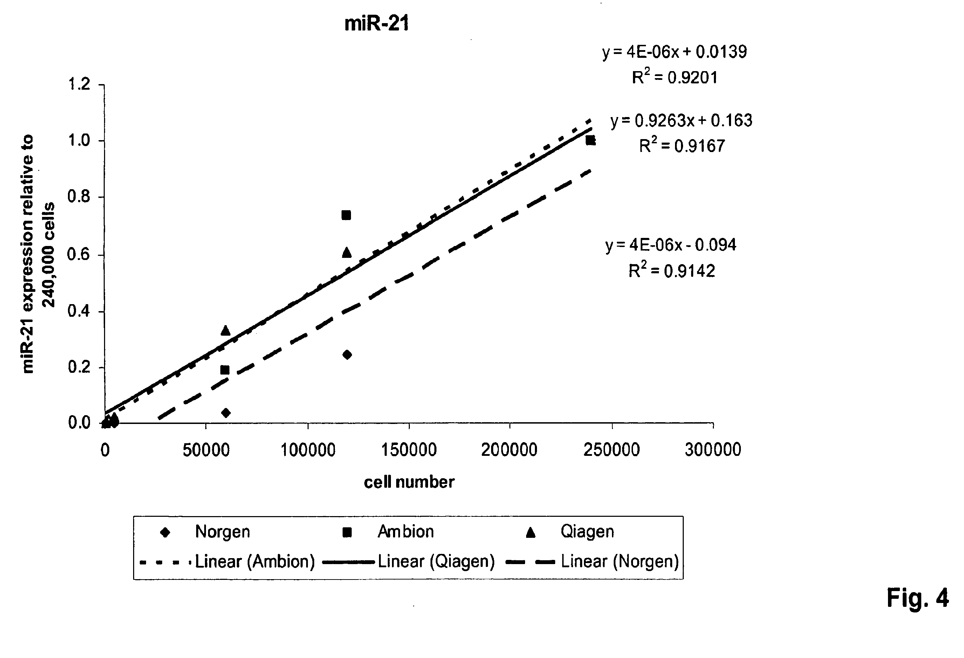

While there are several kits allowing the extraction of mRNA and DNA from several sources, there are currently only three commercial kits allowing the extraction of DNA, mRNA, and miRNAs (QIAGEN miRNeasy Mini Kit, Ambion RecoverAll Total Nucleic Acid Isolation Kit, Norgen All-in-One Purification Kit). First, the extraction capabilities (especially the quantitative recovery of miRNAs) were tested with dilution series of GripTite™ 293 MSR cells (Invitrogen Corp., Carlsbad, CA) ranging from 480 to 240,000 cells. RNA was extracted from the cells according to the respective manufacturer's instructions. Subsequent to extraction, the RNA was reverse transcribed using the QIAGEN miScript Reverse Transcription Kit according to the manufacturer's instructions. Afterwards a miRNA housekeeping RNA (RNU6B), and a further miRNA (miR-21) were quantified on a Roche Lightcycler 480 using the QIAGEN miScript Primer Assays (RNU6B: catalog number: MS00029204, miR-21: catalog number: MS00009079) according to the manufacturer's instructions. The results of these quantifications are shown in Figure 3 and 4. While there are no significant differences for the correlation coefficient of miR-21 expression and the cell number (Figure 4) for the three different extraction methods used there are strong differences for the correlation coefficients for RNU6B expression. The kit showing the best correlation for RNU6B expression in relation to the cell number is the QIAGEN miRNeasy Mini Kit (R²=0.92). Therefore, this kit was used for all extractions.

Subsequently, 20 FNAB slides (10 PTCs, 10 goiters) were used to further evaluate the best performing extraction kit (QIAGEN miRNeasy Mini Kit) for its efficiency regarding DNA and m/miRNA recovery.
In detail, the co-extraction of DNA and RNA from FNAB samples was done as follows. First, the FNAB slides were incubated in Xylol for 4-5 d to remove the cover slips. Afterwards, the slides were air dried. Subsequent, 700 µl Qiazol^{™} (Phenol and Guanidiniumthiocyanat containing lysis reagent by Qiagen, Hilden, Germany) were added to the slide according to the miRNeasy kit (Qiagen, Hilden, Germany) protocol and the cells on the slide were lysed within the Qiazol^{™} using a scalpel. The lysed cells were transferred to a new tube, homogenized by pipetting up and down/vortexing. 240 µl Chloroform were added, mixed for 15 sec and subsequently incubated at room temperature (RT) for 3 min. Then, centrifuge at full speed at 4°C for 15 min. The upper phase was transferred to a new tube and extraction was continued according to the miRNA kit (Qiagen, Hilden, Germany) protocol. The mi/mRNA was eluted in 40 µl ad.
From the first tube rests of the upper phase were removed and 300 µl 96% Ethanol added. The tube was gently mixed and afterwards centrifuged at 8,000g for 3 min. The supernatant was removed and the pellet was incubated with sodium citrate solution for 30 min. Afterwards, the tube was centrifuged at 8,000g for 3 min and again incubated with sodium citrate solution for 30 min. After a centrifugation at 8,000g for further 3 min at RT the pellet was washed with 70% Ethanol. Subsequent to a further centrifugation the pellet was dried at RT for 15 min and then resuspended in 50 µl TE buffer (10 mmol/l Tris-Cl, pH 7.5. 1 mmol/l EDTA). After freezing the DNA for 24 hours it was thawed, vortexed and centrifuged at max. speed for 1 min. The supernatant containing the DNA was transferred to a new tube.

### 2. Quantification of mRNA/miRNA

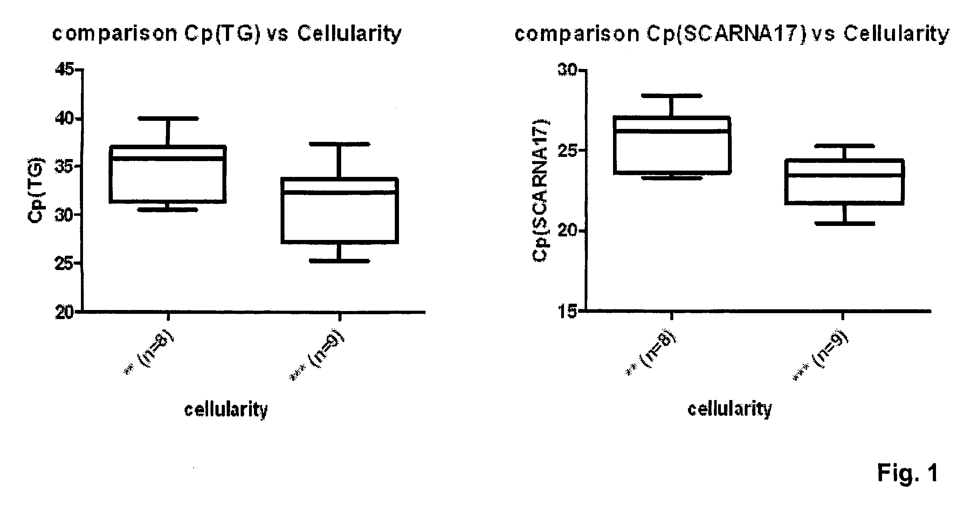

To check the m/miRNA quality and recovery, Thyroglobulin (TG) mRNA as well as the small housekeeping genes were quantified by real time PCR on a Roche LightCycler480 and the results were compared with the number of thyroid cells graded by the pathologist. TG mRNA could be amplified in 19 out of the 20 test FNAB slides and the small housekeeping RNAs could be amplified in all 20 test FNAB samples. Furthermore a correlation of the expression of TG mRNA and the small housekeeping RNAs and the cellularity (++ = 20 - 50 thyroid epithelial cells, +++ > 50 thyroid epithelial cells) of the test samples could be shown (Figure 1).
The quantification of TG mRNA by real time PCR was performed using a LightCycler 480 (Roche, Mannheim, Germany). Oligonucleotide primers were designed to be intron spanning and were purchased from MWG Biotech AG (Ebersberg, Germany). Sequences were obtained from the GenBank database. The nucleotide sequences of the two primers are:
TG-Forward: 5'-CCTGCTGGCTCCACCTTGTTT-3' (SEQ ID No. 1) and
TG-Reverse: 5'-CCTTGTTCTGAGCCTCCCATCGTT-3'(SEQ ID No. 2).

PCRs were performed using the LightCycler DNA Master SYBR Green I Kit (Roche, Mannheim, Germany) according to the manufacturer's instructions. The TG-PCR was processed through 45 cycles including 5 sec of denaturation at 95°C, a 7 sec annealing phase at 62°C and an elongation phase at 72°C for 7 sec. A 20 µl reaction consisted of 2 µl LightCycler FastStart DNA Master SYBR Green I (containing Taq DNA Polymerase, reaction buffer, dNTP mix (with dUTP instead of dTTP) and 10 mmol/l MgCl₂), additional 1.6µl MgCl₂, 0.5 µM of each primer, and 2 µl of template.
Moreover, miR-221 which is known to be up-regulated in PTC, was quantified in the 20 FNAB slides. miR-221 has the following Sequence (Entrez Gene ID 407006):
5'-AGCUACAUUGUCUGCUGGGUUUC-3'(SEQ ID No. 3).

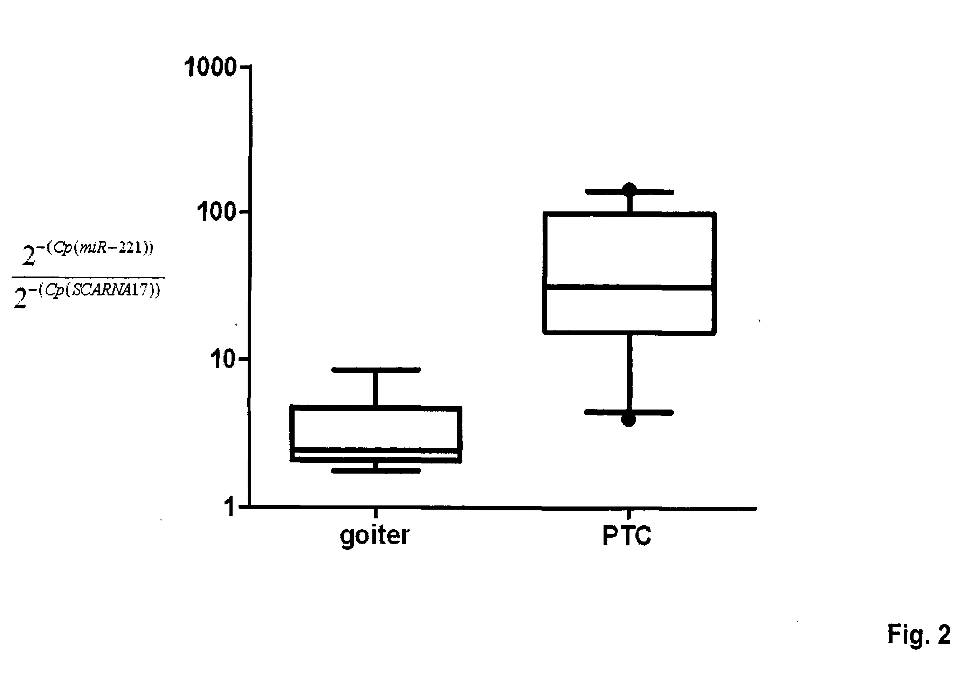

MiR-221 showed a significantly increased expression (p<0.001) in the PTC-FNAB samples compared to the goiter-FNAB samples confirming the data known from the literature and suggesting a successful quantification of miRNAs in RNA samples from FNABs (Figure 2).

DNA was extracted from the left-over of the m/miRNA extraction and DNA quality was checked by amplifying a BRAF fragment, which was possible in all 20 samples. The quantification of BRAF genomic DNA (gDNA) by real time PCR was performed using a LightCycler 480 (Roche, Mannheim, Germany). Primer sequences for the amplification of BRAF were BRAF-F: 5'- TCATAATGCTTGCTCTGATAGGA-3' (SEQ ID No.4) and BRAF-R: 5'-GGCCAAAAATTTAATCAGTGGA-3' (SEQ I D No. 5) according to Nikiforov et al. 2009 (J Clin Endocrinol Metab 94(6):2092-2098). PCRs were run using the LightCycler DNA Master SYBR Green I Kit (Roche, Mannheim, Germany) according to the manufacturer's instructions. The BRAF-PCR was processed through 45 cycles including 5 sec of denaturation at 95°C, a 7 sec annealing phase at 55°C and an elongation phase at 72°C for 9 sec. A 20 µl reaction consisted of 2 µl LightCycler FastStart DNA Master SYBR Green I (containing Taq DNA Polymerase, reaction buffer, dNTP mix (with dUTP instead of dTTP) and 10 mmol/l MgCl₂), additional 1.6µl MgCl₂, 0.5 µmol/l of each primer, and 2 µl of template.

### 3. Detection of RET/PTC and PAX8/PPARG in routine FNAB slides

100 routine air dried FNAB slides from patients who underwent surgery for thyroid nodules at the Odense University Hospital were retrospectively included into this study. In addition, 100 patient matching formalin-fixed paraffin embedded (FFPE) slices were analyzed. All FNAB samples were graded according to the ATA 2006 guidelines by two pathologists. In detail, cytologic evaluation of the FNAB slides revealed 28 malignant, 55 indeterminate, 16 non-neoplastic, and 1 non-diagnostic samples. Histologic evaluation of the corresponding FFPE samples revealed 45 follicular adenoma (FA), 10 oncocytic FA, 4 follicular thyroid carcinoma (FTC), 6 oncocytic FTC, 22 papillary thyroid carcinoma (PTC), 3 follicular variants of PTC (fvPTC), and 10 goiters.

RNA was extracted and reverse transcribed from routine air dried FNAB smears as described above. cDNA was synthesized using the miScript Reverse Transcription Kit (Qiagen, Hilden, Germany) according to the manufacturers suggestions. In brief, 7.5 µl template RNA were added to a master mix consisting of 2 µl 5x miScript RT buffer and 0.5 µl miScript Reverse Transcriptase Mix and incubated for 60 min at 37°C. Subsequently, the miScript Reverse Transcriptase Mix was inactivated for 5 min at 95°C. To check the RNA quality, an intron-spanning 122 bp fragment of *PAX8* mRNA (exon 5-6) was analyzed by real time PCR with subsequent fluorescence melting curve analysis on a Roche LightCycler 480 using FastStart SYBR Green Master chemistry (Roche, Mannheim, Germany). *PAX8*/*PPARG*, *RET*/*PTC1* and *RET*/*PTC3* rearrangements were detected by real time PCR using previously described primers and probes flanking the fusion points (Algeciras-Schimnich A et al.. 2010 Clin Chem 56(3):391-398 and Nikiforov YE et al. 2009. J Clin Endocrinol Metab 94(6):2092-2098) (Table 1) and the LightCycler FastStart DNA MasterPlus HybProbe chemistry (Roche, Mannheim, Germany). These primer/probe combinations allow to detect all four described translocations between *PAX8* and *PPARG: PAX8*(exon1-8)/*PPARG* (PAXB-E8-F/PPARG-E1-R, 85 bp), *PAX8*(exon1-9)/*PPARG* (PAX8-E9-F/PPARG-E1-R, 115 bp), *PAX8*(exon1-10)/*PPARG* (PAX8-E10-F/PPARG-E1-R, 95 bp), and *PAX8*(exon1-8,10)/*PPARG* (PAX8-E10-F/PPARG-E1-R, 188 bp). Moreover, *RET*/*PTC1* (RET/PTC1-F/RET/PTC1-R, 136 bp) and *RET*/*PTC3* (RET/PTC3-F/ RET/PTC3-R, 106 bp) could be detected. PCRs were processed through an initial denaturation at 95°C for 5 min followed by 50 cycles of a 3-step PCR, including 10 sec of denaturation at 95°C, a 10 sec annealing phase at 62°C (*PAX8*/*PPARG*) or 64°C (*RET*/*PTC*) and an elongation phase at 72°C for 7 seconds. cDNA from patient specimens known to carry *PAX8*/*PPARG* or *RET*/*PTC* rearrangements were used as positive controls in each analysis. Positive tested samples were analyzed by capillary gel electrophoresis using BigDye Terminator Kit on an ABI 3100 Genetic Analyzer (Applied Biosystems).

**Table 1. Primers for the detection of RET/PTC and PAX8/PPARG rearrangements and the PCR control PAX8**

| Primer | GenBank ID | sequence | SEQ ID No. |
|---|---|---|---|
| PAX8-E5-F | NM_003466 | TCAACCTCCCTATGGACAGC | 6 |
| PAX8-E6-R | NM_003466 | GGAGTAGGTGGAGCCCAGG | 7 |
| PAX8-E8-F | NM_003466 | CCTCTCGACTCACCAGACCT | 8 |
| PAX8-E9-F | NM_003466 | GCCCTTCAATGCCTTTCCCCATG | 9 |
| PAX8-E10-F | NM_003466 | AGCGGACAGGGCAGCTATGC | 10 |
| PPARG-E1-R | NM_138712 | CCAAAGTTGGTGGGCCAGAAT | 11 |
| PPARG-Probe | NM_138712 | FAM-CATGGTTGACACAGAGAT-BHQ1 | 12 |
| RET/PTC1-F | NM_005436 | GGAGACCTACAAACTGAAGTGCAA | 13 |
| RET/PTC1-R | NM_020975 | CCCTTCTCCTAGAGTTTTTCCAAGA | 14 |
| RET/PTC1-Probe | NM_005436 | FAM-AACCGCGACCTGCGCAAAGC-BHQ1 | 15 |
| RET/PTC3-F | NM_005437 | CCAGTGGTTATCAAGCTCCTTACA | 16 |
| RET/PTC3-R | NM_020975 | GGGAATTCCCACTTTGGATCCTC | 17 |
| RET/PTC3-Probe | NM_005437 | FAM-ACCCAGCACCGACCCCCAGG-BHQ1 | 18 |

| | | | |
|---|---|---|---|
| FAM: Fluorescein; BHQ1: Black Hole Quencher 1. | | | |

Three FNAB samples were tested positive for RET/PTC1 and confirmed by Sanger sequencing. One of these samples was a PTC whose FFPE sample was also RET/PTC1 positive. Two FNAB positive samples are histologically FA and the rearrangement could not be detected in the FFPE samples. One PTC sample was tested positive for RET/PTC3 both in the FNAB and the FFPE sample. Moreover, one further RET/PTC3 rearrangement was detected in a FFPE sample of a FA but the corresponding FNAB sample was mutation negative.

*PAX8*/*PPARG* was detected by RT-PCR in 7 of 76 FFPE samples (9%) and in 6 of 76 FNAB smear samples. In 4 samples it was possible to match FFPE to FNAB (3 FA and 1 FTC). 3 rearrangement positive FFPE cases could not be detected in FNAB, and 2 positive smear samples could not be identified in the corresponding FFPE. PAX8/PPARG was present in 2 of 6 (33%) FTC; 5 of 29 follicular adenomas (17%) and also in 1 Hurthle cell adenoma (n = 8 in total). No rearrangement was detected in Hurthle cell carcinomas (n=1), goiter (n=7) or PTC (n=25). The most frequent fusion variant was PAX8 exons 1-8 juxtaposed to PPARg exon 1 (55%), followed by PAX8 exons 1-9 juxtaposed to PPARg exon 1. The least frequent variant was PAX8 exons 1-10 juxtaposed to PPARg exon 1(16.7%).

These results demonstrate the feasibility of extracting RNA from routine air dried FNA smears to detect PAX8/PPARg rearrangements with RT-PCR. The introduction of molecular analyses of routine air dried FNA smears in every day practice, comprising also other mutations, could provide substantial improvements for the diagnosis of thyroid cancer and thereby potentially also reduce the rate of diagnostic surgery.

### 4. Detection of BRAF, H-, K-, and NRAS mutations in routine FNAB and FFPE slides using hybridization probes

DNA extracted from the FNAB and FFPE samples was screened for the point mutations BRAF V600E and K601 E, and for point mutations in KRAS codons 12/13, and NRAS codon 61 by real time PCR using hybridization probes and fluorescence melting curve analysis on a Lightcycler 480 according to Nikiforov YE et al. 2009 (cited above). The PCRs for the detection of these point mutations were applicable to our DNA samples, which are (due to the extraction from routine FNAB and FFPE samples) of lower quality than the DNAs extracted from fresh FNAB material.

50 FNAB and FFPE samples have been screened for the BRAF V600E mutation using hybridization probes and fluorescence melting curve analysis:

**Table 2:**

| *BRAF* | FNAB | FFPE |
|---|---|---|
| positive in mutation screening | 5 | 10 |
| wildtyp in mutation screening | 15 | 32 |
| questionable | 10 | 4 |
| non-diagnostic (no PCR-product / low efficiency PCR) | 20 | 4 |

In summary, a BRAF mutation could be detected in 29% of the PTC-FNAB samples and in 47% of the PTC-FFPE samples. However, the BRAF detection in the FNAB samples can further improved by High Resolution Melting (HRM) analysis to reduce the number of questionable and non-diagnostic FNAB samples:
The same 50 FNAB and FFPE samples screened by hybridization probes according to Nikiforov et al. were screened by HRM analysis again. PCRs were processed through an initial denaturation at 95°C for 10 min followed by 50 cycles of a 3-step PCR, including 3 sec of denaturation at 95°C, a 10 sec annealing phase at 58°C and an elongation phase at 72°C for 10 seconds on a LightCycler 480. Subsequently a high resolution melting curve was assessed. The following primers were used:
   BRAF-F: 5' -GGTGATTTTGGTCTAGCTACAG-3' (SEQ ID No. 19) and
   BRAF-R:5'-GGCCAAAAATTTAATCAGTGGA-3' (SEQ ID No. 20).

The results of the HRM assay are as follows:

**Table 3:**

| *BRAF* | FNAB | FFPE |
|---|---|---|
| positive in mutation screening | 9 | 12 |
| wildtyp in mutation screening | 40 | 35 |
| questionable | 0 | 3 |
| non-diagnostic (no PCR-product / low efficiency PCR) | 1 | 0 |

The comparison of the two methods shows that HRM analysis detects more *BRAF* mutations (FNAB: 9 vs. 5, FFPE: 12 vs. 10) and results in less questionable/non-diagnostic results (FNAP: 0/1 vs. 10/20, FFPE: 3/0 vs. 4/4). In summary, a *BRAF* mutation could be detected in 29% of the PTC-FNAB samples by using hybridization probes and 47% of the PCT-FNAB samples by HRM. Furthermore, a *BRAF* mutation could be detected in 47% of the PTC-FFPE samples by using hybridization probes and 70% of the PCT-FFPE samples by HRM. The occurrence of mutations was verified by Sanger sequencing. These results clearly indicate that HRM is superior to the use of hybridization probes in detecting *BRAF* point mutations.

So far NRAS-screening revealed that 3 FA of all screened FNAB-samples carried a NRAS-mutation, whereas 15 (22.7%) of the FFPE-samples were tested positive for NRAS mutation.

**Table 4:**

| NRAS | FNAB | FFPE |
|---|---|---|
| positive in mutation screening | 3 | 15 |
| wildtyp in mutation screening | 53 | 47 |
| non-diagnostic (no PCR-product / low efficiency PCR) | 10 | 4 |

NRAS mutations can also be detected in an High Resolution Melting (HRM) assay as described for BRAF and KRAS.

Up to now 70 samples were screened for point mutations in KRAS codons 12/13 using hybridization probes PCR. No KRAS-mutation was thus far detected in FNAB- and FFPE samples and approximately three-third of all screened FNAB- and FFPE-samples were WT. Compared with the 11 non-diagnostic FNAB-samples (15.7%) that showed no or a weak amplification in the PCR, there where 10 FFPE-samples (14.2%) with the same problems.

**Table 5:**

| KRAS | FNAB | FFPE |
|---|---|---|
| positive in mutation screening | 0 | 0 |
| wildtyp in mutation screening | 53 | 59 |
| questionable | 6 | 1 |
| non-diagnostic (no PCR-product / low efficiency PCR) | 11 | 10 |

A High Resolution Melting (HRM) assay was established also to detect KRAS mutations and the results of both methods were compared.
The same 70 FNAB and FFPE samples screened by hybridization probes according to Nikiforov et al. 2009 (cited above) were screened by HRM analysis again. PCRs were processed through an initial denaturation at 95°C for 10 min followed by 50 cycles of a 3-step PCR, including 3 sec of denaturation at 95°C, a 12 sec annealing phase at 58°C and an elongation phase at 72°C for 10 seconds on a Lightcycler 480. Subsequently a high resolution melting curve was assessed. The following primers were used:
KR.AS-F: 5' - AGGCCTGCTGAAAATGACTG -3' (SEQ ID No. 21) and
KRAS-R:5'- GCTGTATCGTCAAGGCACTCT -3' (SEQ ID No. 22).

The results of the HRM assay are as follows:

**Table 6:**

| KRAS | FNAB | FFPE |
|---|---|---|
| positive in mutation screening | 3 | 1 |
| wildtyp in mutation screening | 55 | 48 |
| questionable | 0 | 13 |
| non-diagnostic (no PCR-product / low efficiency PCR) | 12 | 8 |

In summary, the experiments show that the PCRs using hybridization probes for mutation detection could be applied also to DNAs of lower quality. High resolution melting (HRM) analysis for detection of the point mutations is preferred.

*5. Liquid cytology - Quantification of TG and RPL27 mRNA in relation to the amount of liquid based FNAB material*For liquid based cytology the material obtained by FNAB is expelled into a cell preservation solution (methanol based cytolyt^{™} solution, Cytyc Corp. Marlborough, MA, USA). Thereafter, the cells are spun and the pellet is transferred into preservCyt^{™} (Cytyc Corp) for further processing in the T2000 automated processor (Cytyc Corp) according to the manufacturer's recommendations. A thin evenly dispersed monolayer of cells was dispersed from the filter onto the slide in a cycle of 20 mm in diameter (ThinPrep slides). ThinPrep slides were stained and histology was performed.
The cells not used for making the slide are stored in the cell preservation solution e.g. preserveCyt TM and subsequently used for parallel RNA (in particular mRNA and miRNA) and DNA extraction. Alternatively, RNA and DNA extraction is performed with the cells removed from the slide after staining and histology.
For RNA ad DNA extraction the samples were transferred to Falcon tubes and pelleted by centrifugation. Afterwards, the supernatant was removed and 700 µl Qiazol (Qiagen, Hilden, Germany) were added to the pellet according to the miRNeasy kit protocol and the cells were lysed within the Qiazol. The lysed cells were transferred to a new tube, homogenized by pipetting up and down/vortexing. 240 µl Chloroform were added, mixed for 15 sec and subsequently incubated at room temperature for 3 min. Then, the samples were centrifuged at full speed at 4°C for 15 min. The upper phase was transferred to a new tube and extraction was continued according to the miRNA kit protocol. The mi/mRNA was eluted in 40 µl ad.
From the first tube rests of the upper phase were removed and 300 µl 96% Ethanol added. The tube was gently mixed and afterwards centrifuged at 8,000g for 3 min. The supernatant was removed and the pellet was incubated with sodium citrate solution for 30 min. Afterwards, the tube was centrifuged at 8,000g for 3 min and again incubated with sodium citrate solution for 30 min. After a centrifugation at 8,000g for further 3 min at room temperature the pellet was washed with 70% Ethanol. Subsequent to a further centrifugation the pellet was dried at room temperature for 15 min and then resuspended in 50 µl TE buffer. After freezing the DNA for 24 hours it was thawed, vortexed and centrifuged at max. speed for 1 min. The supernatant containing the DNA was transferred to a new tube.

The amount of liquid based FNAB material supplied was documented as "+ - minimal amount of material visible" to "+++++ - very high amount of material". DNA and RNA was extracted from all samples. To check the extracted RNA and DNA, the RNA was reverse transcribed (as described above) and TG (as described above) and the housekeeping gene RPL27 were quantified by real time PCR. Moreover, using the extracted DNA BRAF was amplified and checked for the BRAF V600E mutation by HRM (as described above).
The RPL27-PCR was processed through 45 cycles including 5 sec of denaturation at 95°C, a 7 sec annealing phase at 60°C and an elongation phase at 72°C for 9 sec. A 20 µl reaction consisted of 2 µl LightCycler FastStart DNA Master SYBR Green I (containing Taq DNA Polymerase, reaction buffer, dNTP mix (with dUTP instead of dTTP) and 10 mmol/l MgCl₂), additional 1.6µl MgCl₂, 0.5 µmol/l of each primer, and 2 µl of template. The following primers were used:
RPL27-F: 5'-ATCGCCAAGAGATCAAAGATAA-3' (SEQ ID No. 23) and
RPL27-R: 5'- TCTGAAGACATCCTTATTGACG-3'(SEQ ID No. 24).

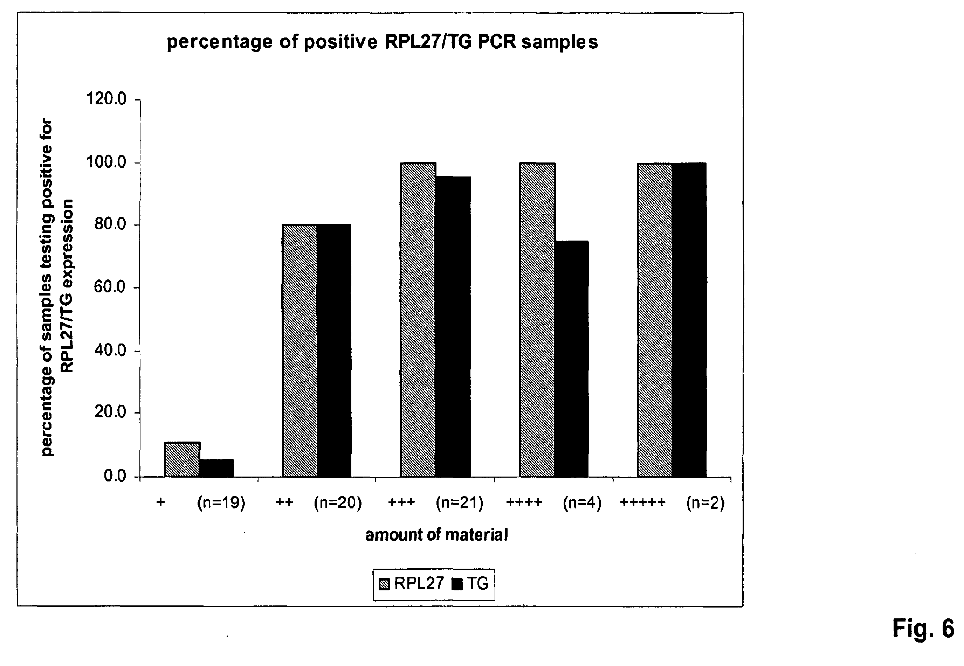

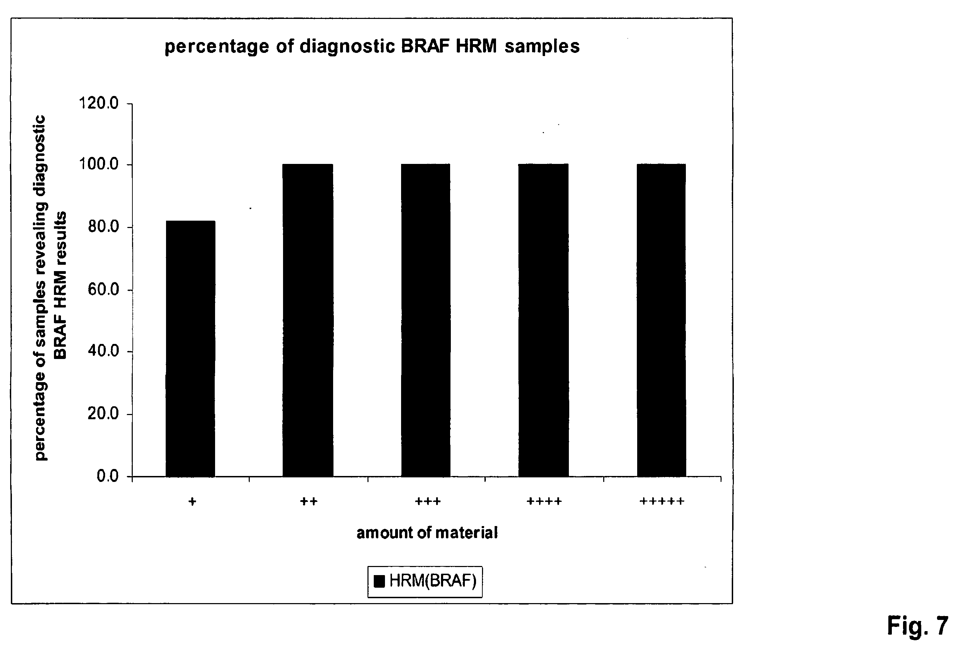

Both, RPL27 and TG mRNA could be amplified in at least 80% of samples providing at least 1ml of material (++) (Figure 6). Moreover, a screening for BRAF mutations by HRM was possible in all samples providing at least 1 ml of material (Figure 7).

## Claims

1. A method for distinguishing malignant from benign tumor samples of the thyroid, by
a.) performing an RNA extraction in an air dried and/or fixed fine needle aspiratipn biopsy (FNAB) sample, wherein RNA isolation is performed after cytological fixation and staining of the air dried and/or fixed fine needle aspiration biopsy (FNAB) sample,
b.) analyzing the presence of gene-rearrangements and/or differential expression of miRNA in the isolated RNA, wherein the presence of a gene-rearrangement and/or the differential expression of miRNA is indicative for a malignant tumor.

2. A method according to claim 1 wherein in step b.) rearrangements of *RET*/*PTC* and/or P*AX8*/*PPARG* (paired box 8/peroxisome proliferator-activated receptor gamma) are analyzed.

3. A method according to claim 1 or 2 wherein the analysis in step b.) is carried out by RT-PCR or Pyrosequencing.

4. A method according to one of the claims 1 to 3 wherein in step a.) mRNA, miRNA and DNA are extracted simultaneously from the fine needle aspiration biopsy tumor sample.

5. A method according to claim 4 wherein point mutations in DNA and/or miRNA are analyzed additionally, and wherein the presence of a point mutation in the isolated DNA and/or miRNA is indicative for a malignant tumor.

6. A method according to claim 5 wherein point mutations in DNA encoding *BRAF*, *N*-, *K*-, and/or *HRAS* are analyzed.

7. A method according to one of the claims 1 to 6 wherein the analyzed miRNA is miR-21, miR-181, miR-182, miR-187, miR-221 and/or miR-222

8. A method according to claim 7 wherein the analyzed miRNA is preferably miR-221.

9. A method according to one of the claims 1 to 8 wherein the presence of RET/PTC and/or PAX8/PPARG rearrangements classifies the tumor as malignant.

10. A method according to one of the claims 1 to 9, wherein the fine needle aspiration biopsy (FNAB) sample is a FNAB smear or a liquid based cytology sample.

11. In vitro use of a kit comprising at least one of the following:
i. Buffer for nucleic acid extraction,
ii. Primer, buffers and RNA-dependent-DNA-polymerase for Reverse transcription,
iii. Primers, buffers, dNTP mix, and a DNA-Polymerase for PCR on cDNA
for performing a method according to one of the claims 1 to 10.

12. In vitro use of a kit according to claim 11 comprising additionally one or more of the following:
i. Primers, buffers, dNTP mix, and a DNA-Polymerase for PCR on genomic DNA,
ii. oligonucleotide probes (preferably single or dual Fluorescence-labeled) for the detection of rearrangements and/or miRNA,
iii. sequencing primers.

13. In vitro use of a kit comprising at least one of the following:
i. Buffer for nucleic acid extraction,
ii. Reagents for labeling RNA and/or DNA,
iii. Hybridization buffers,
iv. Washing buffers
for performing a method according to one of the claims 1 to 10.

## Patentansprüche

1. Ein Verfahren zur Unterscheidung bösartiger von gutartigen Tumor-Proben der Schilddrüse, mittels
a) Durchführen einer RNA-Extraktion in einer luftgetrockneten und/oder fixierten Feinnadelaspirationsbiopsie (FNAB) - Probe, wobei die RNA-Isolation nach der zytologischen Fixierung und Färbung der luftgetrockneten und/oder fixierten Feinnadelaspirationsbiopsie (FNAB) - Probe durchgeführt wird,
b) Analysieren des Vorhandenseins von Gen-Rearrangements und/oder unterschiedlicher Expression von miRNA in der isolierten RNA, wobei das Vorhandensein von Gen-Rearrangements und/oder unterschiedlicher Expression von miRNA auf einen bösartigen Tumor hinweist.

2. Ein Verfahren nach Anspruch 1, wobei in Schritt b) Rearrangements von *RET*/*PTC* und/oder *PAX8*/*PPARG* (paired box 8/ peroxisome proliferator-activated receptor gamma) analysiert werden.

3. Ein Verfahren nach Anspruch 1 oder 2, wobei die Analyse in Schritt b) mittels RT-PCR oder Pyrosequenzierung durchgeführt wird.

4. Ein Verfahren nach einem der Ansprüche 1 bis 3, wobei in Schritt a) mRNA, miRNA und DNA gleichzeitig aus der Feinnadelaspirationsbiopsie-Tumor-Probe extrahiert werden.

5. Ein Verfahren nach Anspruch 4, wobei Punktmutationen in DNA und/oder miRNA zusätzlich analysiert werden und wobei das Vorhandensein von einer Punktmutation in der isolierten DNA und/oder miRNA auf einen bösartigen Tumor hinweist.

6. Ein Verfahren nach Anspruch 5, wobei Punktmutationen in DNA kodierend für *BRAF, N*-, *K*- und/oder *HRAS* analysiert werden.

7. Ein Verfahren nach einem der Ansprüche 1 bis 6, wobei die analysierte miRNA miR-21, miR-181, miR-182, miR-187, miR-221 und/oder miR-222 ist.

8. Ein Verfahren nach Anspruch 7, wobei die analysierte miRNA bevorzugt miR-221 ist.

9. Ein Verfahren nach einem der Ansprüche 1 bis 8, wobei das Vorhandensein von RET/PCT- und/oder PAX8/PPARG-Rearrangements den Tumor als bösartig einstuft.

10. Ein Verfahren nach einem der Ansprüche 1 bis 9, wobei die Feinnadelaspirationsbiopsie (FNAB) - Probe ein FNAB-Abstrich oder eine Dünnschichtzytologie-Probe ist.

11. *In vitro* Verwendung eines Kits, enthaltend mindestens einen der folgenden Bestandteile :
i. Puffer für Nukleinsäure-Extraktion,
ii. Primer, Puffer und RNA-abhängige DNA-Polymerase für Reverse Transkription,
iii. Primer, Puffer, dNTP-Mix und eine DNA-Polymerase für PCR bei cDNA,
für die Durchführung eines Verfahrens nach einem der Ansprüche 1 bis 10

12. *In vitro* Verwendung eines Kits, nach Anspruch 11, enthaltend zusätzlich einen oder mehrere der folgenden Bestandteile :
i. Primer, Puffer, dNTP-Mix und eine DNA-Polymerase für PCR bei genomischer DNA,
ii. Oligonukleotidsonden (bevorzugt einfach oder doppelt fluoreszenzmarkiert) für die Detektion von Rearrangements und/oder miRNA,
iii. Sequenzierprimer.

13. *In vitro* Verwendung eines Kits, enthaltend mindestens einen der folgenden Bestandteile :
i. Puffer für Nukleinsäure-Extraktion,
ii. Reagenzien zur Markierung von RNA und/oder DNA,
iii. Hybridisierungspuffer
iv. Waschpuffer,
zur Durchführung eines Verfahrens nach einem der Ansprüche 1 bis 10.

## Revendications

1. Procédé pour distinguer des échantillons de tumeur maligne de tumeur bénigne de la thyroïde, en
a) réalisant une extraction d'ARN dans un échantillon de biopsie à aspiration par aiguille fine (FNAB) séché à l'air et/ou fixé, dans lequel l'isolement d'ARN est réalisé après la fixation cytologique et la coloration de l'échantillon de biopsie séché à l'air et/ou à aspiration par aiguille fine fixe (FNAB),
b) analysant la présence de réarrangements de gène et/ou l'expression différentielle de miARN dans l'ARN isolé, dans lequel la présence d'un réarrangement de gène et/ou l'expression différentielle de miARN indique la présence d'une tumeur maligne.

2. Procédé selon la revendication 1 dans lequel dans l'étape b) les réarrangements de *RET*/*PTC* et/ou de *PAX8*/*PPARG* (boîte appariée 8/récepteur gamma activé par les proliférateurs de peroxisome) sont analysés.

3. Procédé selon la revendication 1 ou 2 dans lequel l'analyse dans l'étape b) est réalisée par RT-PCR ou pyroséquençage.

4. Procédé selon l'une des revendications 1 à 3 dans lequel dans l'étape a) les ARNm, les miARN et l'ADN sont extraits simultanément de l'échantillon de tumeur de biopsie à aspiration par aiguille fine.

5. Procédé selon la revendication 4 dans lequel les mutations ponctuelles dans l'ADN et/ou les miARN sont analysés en plus, et dans lequel la présence d'une mutation ponctuelle dans l'ADN et/ou du miARN isolé est indicateur d'une tumeur maligne.

6. Procédé selon la revendication 5 dans lequel les mutations ponctuelles dans l'ADN codant *BRAF, N-, K-* et/ou *HRAS* sont analysées.

7. Procédé selon l'une quelconque des revendications 1 à 6 dans lequel le miARN analysé est miR-21, miR-181, miR-182, miR-187, miR-221 et/ou miR-222.

8. Procédé selon la revendication 7 dans lequel le miARN analysé est de préférence miR-221.

9. Procédé selon l'une des revendications 1 à 8 dans lequel la présence de réarrangements de RET/PTC et/ou de PAX8/PPARG classifie la tumeur comme maligne.

10. Procédé selon une des revendications 1 à 9, dans lequel l'échantillon de biopsie à aspiration par aiguille fine (FNAB) est un frottis de FNAB ou un échantillon de cytologie à base de liquide.

11. Utilisation *in vitro* d'un kit comprenant au moins un de ce qui suit :
i. tampon pour extraction d'acide nucléique,
ii. amorce, tampons, et ADN polymérase dépendante de l'ARN pour la transcription inverse,
iii. amorces, tampons, mélange de dNTP, et une ADN polymérase pour une PCR surADNc
pour mettre en oeuvre un procédé selon une des revendications 1 à 10.

12. Utilisation d'un kit *in vitro* selon la revendication 11 comprenant de plus un ou plusieurs de ce qui suit :
i. amorces, tampons, mélange de dNTP, et une ADN polymérase pour une PCR sur ADN génomique,
ii. sondes oligonucléotidiques (de préférence marquées à simple ou double fluorescence) pour la détection de réarrangements et/ou de miARN,
iii. amorces de séquençage.

13. Utilisation d'un kit *in vitro* comprenant au moins un de ce qui suit :
i. tampon pour extraction d'acide nucléique
ii. réactifs pour marquer l'ARN et/ou l'ADN,
iii. tampons d'hybridation,
iv. tampons de lavage
pour mettre en oeuvre un procédé selon une des revendications 1 à 10.


### SEQUENCE LISTING

<110> Technische universität Dresden
<120> Method and means for distinguishing malignant from benign tumor samples, in particular in routine air dried fine needle aspiration biopsy (FNAB)
<130> 00401P0058EP
<160> 24
<170> PatentIn version 3.5
<210> 1
   <211> 21
   <212> DNA
   <213> Artificial Sequence
<220>
   <223> Primer
<400> 1
   cctgctggct ccaccttgtt t 21
<210> 2
   <211> 24
   <212> DNA
   <213> Artificial Sequence
<220>
   <223> Primer
<400> 2
   ccttgttctg agcctcccat cgtt 24
<210> 3
   <211> 23
   <212> RNA
   <213> Homo sapiens
<400> 3
   agcuacauug ucugcugggu uuc 23
<210> 4
   <211> 23
   <212> DNA
   <213> Artificial Sequence
<220>
   <223> Primer
<400> 4
   tcataatgct tgctctgata gga 23
<210> 5
   <211> 22
   <212> DNA
   <213> Artificial Sequence
<220>
   <223> Primer
<400> 5
   ggccaaaaat ttaatcagtg ga 22
<210> 6
   <211> 20
   <212> DNA
   <213> Artificial sequence
<220>
   <223> Primer
<400> 6
   tcaacctccc tatggacagc 20
<210> 7
   <211> 19
   <212> DNA
   <213> Artificial Sequence
<220>
   <223> Primer
<400> 7
   ggagtaggtg gagcccagg 19
<210> 8
   <211> 20
   <212> DNA
   <213> Artificial Sequence
<220>
   <223> Primer
<400> 8
   cctctcgact caccagacct 20
<210> 9
   <211> 23
   <212> DNA
   <213> Artificial Sequence
<220>
   <223> Primer
<400> 9
   gcccttcaat gcctttcccc atg 23
<210> 10
   <211> 20
   <212> DNA
   <213> Artificial Sequence
<220>
   <223> Primer
<400> 10
   agcggacagg gcagctatgc 20
<210> 11
   <211> 21
   <212> DNA
   <213> Artificial Sequence
<220>
   <223> Primer
<400> 11
   ccaaagttgg tgggccagaa t 21
<210> 12
   <211> 18
   <212> DNA
   <213> Artificial Sequence
<220>
   <223> Primer
<400> 12
   catggttgac acagagat 18
<210> 13
   <211> 24
   <212> DNA
   <213> Artificial Sequence
<220>
   <223> Primer
<400> 13
   ggagacctac aaactgaagt gcaa 24
<210> 14
   <211> 25
   <212> DNA
   <213> Artificial Sequence
<220>
   <223> Primer
<400> 14
   cccttctcct agagtttttc caaga 25
<210> 15
   <211> 20
   <212> DNA
   <213> Artificial Sequence
<220>
   <223> Primer
<400> 15
   aaccgcgacc tgcgcaaagc 20
<210> 16
   <211> 24
   <212> DNA
   <213> Artificial Sequence
<220>
   <223> Primer
<400> 16
   ccagtggtta tcaagctcct taca 24
<210> 17
   <211> 23
   <212> DNA
   <213> Artificial Sequence
<220>
   <223> Primer
<400> 17
   gggaattccc actttggatc ctc 23
<210> 18
   <211> 20
   <212> DNA
   <213> Artificial Sequence
<220>
   <223> Primer
<400> 18
   acccagcacc gacccccagg 20
<210> 19
   <211> 22
   <212> DNA
   <213> Artificial Sequence
<220>
   <223> Primer
<400> 19
   ggtgattttg gtctagctac ag 22
<210> 20
   <211> 22
   <212> DNA
   <213> Artificial Sequence
<220>
   <223> Primer
<400> 20
   ggccaaaaat ttaatcagtg ga 22
<210> 21
   <211> 20
   <212> DNA
   <213> Artificial Sequence
<220>
   <223> Primer
<400> 21
   aggcctgctg aaaatgactg 20
<210> 22
   <211> 21
   <212> DNA
   <213> Artificial Sequence
<220>
   <223> Primer
<400> 22
   gctgtatcgt caaggcactc t 21
<210> 23
   <211> 22
   <212> DNA
   <213> Artificial Sequence
<220>
   <223> Primer
<400> 23
   atcgccaaga gatcaaagat aa 22
<210> 24
   <211> 22
   <212> DNA
   <213> Artificial Sequence
<220>
   <223> Primer
<400> 24
   tctgaagaca tccttattga cg 22